(19) **Europäisches Patentamt / European Patent Office / Office européen des brevets**

(11) **EP 1 485 155 B1**

(12) **EUROPEAN PATENT SPECIFICATION**

(45) Date of publication and mention
of the grant of the patent:
**21.02.2007 Bulletin 2007/08**

(51) Int Cl.:
***A61M 16/00*** (2006.01)

(21) Application number: **03712519.2**

(86) International application number:
**PCT/IB2003/001392**

(22) Date of filing: **10.03.2003**

(87) International publication number:
**WO 2003/075989 (18.09.2003 Gazette 2003/38)**

(54) **AN AIR ASSISTANCE APPARATUS PROVIDING FAST RISE AND FALL OF PRESSURE WITHIN ONE PATIENT S BREATH**

LUFTUNTERSTÜTZUNGSAPPARAT FÜR SCHNELLEN DRUCKANSTIEG UND -ABSENKUNG WÄHREND EINES ATEMZUGES EINES PATIENTEN

APPAREIL D'ASSISTANCE RESPIRATOIRE PRODUISANT UNE AUGMENTATION ET UNE CHUTE RAPIDE DE LA PRESSION DE L'AIR RESPIRE PAR LE PATIENT

(84) Designated Contracting States:
**AT BE BG CH CY CZ DE DK EE ES FI FR GB GR HU IE IT LI LU MC NL PT RO SE SI SK TR**

(30) Priority: **08.03.2002 US 362441 P**

(43) Date of publication of application:
**15.12.2004 Bulletin 2004/51**

(73) Proprietor: **Kaerys S.A.**
**06300 Nice (FR)**

(72) Inventors:
• **DELACHE, Alain**
**F-06300 Nice (FR)**

• **DELACHE, Véronique**
**F-06300 Nice (FR)**

(74) Representative: **de Zeeuw, Johan Diederick et al**
**Murgitroyd & Company,**
**Immeuble Atlantis**
**55, Allée Pierre Ziller,**
**Sophia Antipolis**
**06560 Valbonne (FR)**

(56) References cited:
**WO-A-02/26305          US-A- 5 977 737**
**US-A1- 2002 000 228**

**Description**

Technical field

The present invention concerns a medical apparatus to assist a patient respiration and more specifically an apparatus able to control the pressure of the air provided to the patient.

Background art

Some apparatus are used to provide patients with air. This is used in many treatments. More frequently it is used for patients with a breathing deficiency caused for example by the weakness of the breathing system or by obstructive apneas during the sleep. In those cases it is important to control the pressure of the air delivered to the patient. With respiratory insufficient patients, apparatus providing air at a higher pressure help to compensate the weakness of the patients lungs. In the case of patients suffering of sleep apneas, providing the air at a higher pressure removes the obstruction of the upper airways.

Several kinds of apparatus are used. The CPAP (Continuous Positive Airway Pressure) apparatus provides a constant pressure to the patient. An enhancement of the CPAP is adjusting the pressure delivered according to the detection of events (snoring, apneas, hypopneas,...) for example, those apparatus will increase pressure (within bounds) if events are detected and will reduce pressure (within bounds) if no events are detected during a defined duration. Other apparatus operate with two different levels of pressure, one for the inspiration phase, the other one for the expiration phase. They treat the sleep apnea disorder as well as some respiratory insufficient patients. Those apparatus are composed of a centrifugal blower which provides the air. This blower comprises an impeller which is rotated by an electrical motor using the brushless technology. The rotor is a multi poles permanent magnet and the stator is an electrical magnet with several crenels each of them having a winding. The windings are alternatively connected to a power source so that the magnetic field changes in the stator crenels enable the rotor to rotate.

These apparatus require efficient motors. In order to operate sensors are precisely positioned on the stator to detect the position of the rotor. The sensors are connected to a processor in order to provide the information concerning the position of the rotor. The processor is then able to drive the windings phases according to the rotor position. The efficiency of those motors is highly linked on the precision of the positioning of the sensors and the reliability of the system is linked to the reliability of the sensors. Those sensors detect the position of the rotor by the variations of the magnetic flow and thus their reliability depends also on their bandwidth. Standard sensors have usually a 10 kHz bandwidth and thus are not able to operate correctly at high speeds. Higher speeds can be provided by using sensors with a higher bandwidth but those sensors are expensive and still have a speed limitation. In fact the faster the rotor rotates the worse the detection is accurate, the less the motor is efficient. Sometimes the system can even stall. Because these problems of positioning and reliability, those air providing apparatus which are using these sensors do not use high speed motors. The provided pressure is proportional to the impeller rotation speed and to the size of the impeller. Thus, in order to have the required pressure the size of their impeller is increased. Mainly those apparatus use big impellers having a diameter comprised between 70 and 100 millimeters. Such impellers imply high inertia, thus preventing quick speed changes.

Moreover, in the case of a CPAP that does not require pressure changes within a breath, to have a well regulated pressure at the patient interface, these apparatus are requiring the use of a 22 millimeters diameter tube as the pressure drop within such a tube is negligible but the use of a smaller tube will not guarantee the pressure regulation within acceptable bounds.

In the case of two levels of pressure apparatus, as changing the blower speed within a breath is impossible to achieve due to the high inertia, the highest level is delivered by the blower and the lowest is managed by using pneumatic valves, pressure dividers or alternative methods.

The size of the impeller, the sensor positioning and attachments and the number of wires required increase the size of the blower and thus of the apparatus. Furthermore the precise positioning of the sensors complicates the assembling of the motor implying an increase of the cost.

In US 2002/0000228, the inventors have used a way to obtain fast accelerations and decelerations which does not rely on the positioning of the rotor. US 2002/0000228 discloses a blower for an air assistance apparatus. The blower operates with an electric motor. The polarity of the stator's sectors are changed by a driving unit to achieve fast accelerations and decelerations in one patient's breath step. This result is obtained by using a compressor wheel with a very low mass and by holding the wheel without contact in the radial compressor. This system requires a lot of precision when assembling the motor to obtain the magnetic fields that will hold the wheel without contact in the compressor. The rotor position can not be determined.

Document US 5977737 relates to a way to enhance the accuracy of the motor functions. The improvement of the accuracy is carried out by predicting the motor current. There are no means for sensing the motor current. The prediction of the motor current is made by using different parameters such as motor inductance, motor and driver resistance and the back-electo motor force (back-EMF) value. The motor's position may also be included as one of the parameters. Using these parameters the current is calculated. This allows the determination of the pulse widths to be applied to various inputs to the driver circuit. The back-EMF is not used to determine the rotor's position and to change the sector's polarity in respect of this position. US 5977737 does not disclose a way to determine the rotor's position. The aspect disclosed by US 5977737 is a more accurate delivery of current and not a way for increasing the electro motor's efficiency. This document thus does not provide any indication to obtain fast accelerations and decelerations within a patient's breath.

Summary of the invention

The invention object is to provide an apparatus able to deliver air to a patient at a controlled pressure in such a way that the pressure could be modulated within a breath according to the breath pattern and according to events detected from the patient.

Another object is to provide an apparatus enabling the use of smaller diameter tubes than the standard 22mm.

Another object of the invention is to provide an apparatus with a lower size, being more power efficient and to improve comfort, to be as quiet as possible.

A further object is to provide an apparatus easier and cheaper to assemble.

The subject of the invention is an apparatus as defined in claim 1 able to deliver air to a patient at a controlled pressure in such a way that the pressure could be modulated within a breath according to the breath pattern and according to events detected from the patient.

This is obtained by a very efficient centrifugal blower which is able to rotate at high speed (up to 50000 round per minute) and to decelerate and accelerate very quickly ($\pm$10 hPa in 30 milliseconds). This efficiency is obtained by having a very light and small diameter impeller, preferentially 50mm, and having preferentially a weight of 10 grams. This efficiency is obtained in the motor by detecting the optimal rotor position by measuring the back electro motor force (BEMF) generated in the blower motor. In respect of the BEMF detected a computer or processor is able to know the position of the rotor and accordingly will change the phases of the stator branches.

The use of this technology avoids using sensors which enable the rotor to rotate at high speeds without malfunctioning. Tubes of a 15 millimeters can be used without pressure drops. The small size of the blower enables to reduce the whole apparatus and enables to create an ambulatory apparatus.

An other implementation of this technology is that a toroidal stator is used. This further increases the efficiency of the blower and its size. This toroidal stator is also easiest and cheapest to assemble.

Another side of the invention relates to its ability to communicate with its power supply.

An implementation of this invention is to be able to modulate the pressure of the air provided. This modulation occurring within one inspiration or expiration, which is enabled by the capacity of the apparatus to provide fast accelerations and decelerations.

Also the apparatus is able to react to events occurring in patients breathing and in response to modify the provided air pressure, possibly to adopt a given shape of the pressure to patient's mask being a function of time according to the frequency of the patient's breathing parameters. The apparatus according to present invention has the capacity to provide such a modulation within one single breath.

Brief description of figures

    Figure 1 represents the connections between the elements of the apparatus according to the present invention,
    Figure 2 represents the assembling of the centrifugal blower of the apparatus according to the present invention,
    Figure 3 represents a section of the blower of the apparatus according to the present invention,
    Figure 4a is a symbolic representation of the three stator sectors connected to the power source,
    Figure 4b represents a preferred embodiment of the stator,
    Figure 5 is a symbolic representation of the different switches which connect the stator sectors to the power source,
    Figure 6 is a simplified representation which shows one configuration of switches setting,
    Figure 7 represents of the Pulse Width Modulation in function of time,
    Figure 8 represents the connections which enable to control the rotor rotation,
    Figure 9 represents the process to start the motor functioning,
    Figure 10 represents the communication of the data in the apparatus,
    Figure 11 represents the electric scheme of the load control module of the apparatus according to the present invention,

Figure 12 represents the process of the load control of the apparatus according to the present invention,
Figure 13 represents a starting operating process of the apparatus according to the present invention,
Figure 14 represents the way the tension to apply to the stator is determinate,
Figure 15 represents an example of air pressure applied to the patient in respect of his respiration,
Figure 16a and 16 b represent respectively the patient's breathing pattern and the pattern of the pressure of the air provided by the apparatus according to present invention, and
Figure 17 represents one way the apparatus reacts to an event.

Detailed description of the invention

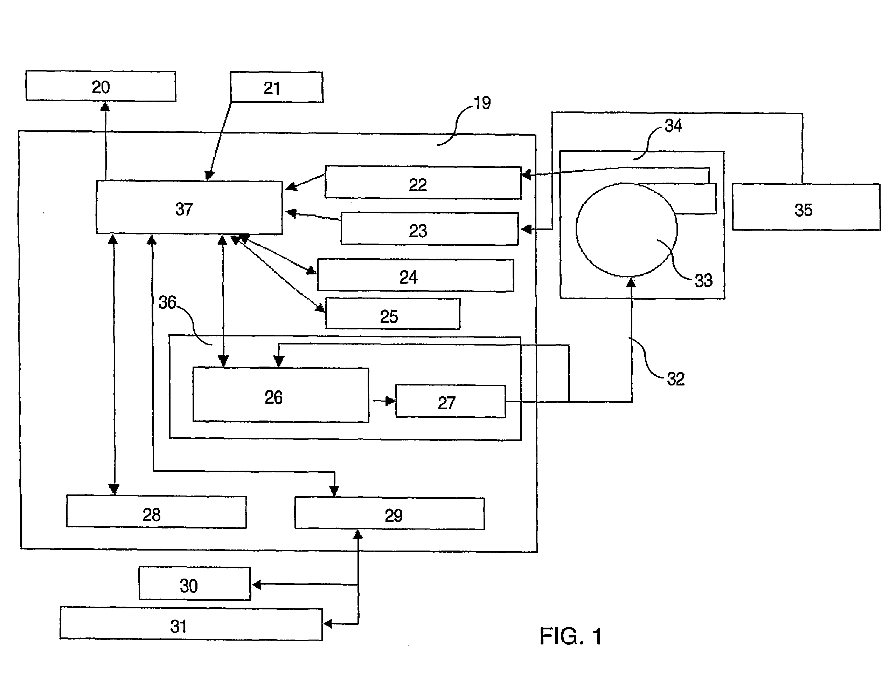

The apparatus according to the present invention as defined in claim 1 provides a patient under treatment with air under controlled pressure. As represented in figure 1, the apparatus includes a high speed and low inertia centrifugal blower 33, an acoustic assembly 34, a computerized electronic board 19, a graphical display 20, a keyboard 21, and a sensor 35 connected to the a tube first extremity, connected to the patient's mask. This tube is connected to the blower 33 on its second extremity, but as the figure 1 represents the alimentation and the communication connections, the tube has not been represented.

In a preferred embodiment, the computerized electronic board 19 includes :

- means 23 and 35 for sensing the pressure at the patient's mask,
- means 22 for sensing the pressure at the centrifugal blower 33,
- a non volatile memory 24 used to store patient compliance data's and measurements,
- a real time clock 25 used to time mark the events,
- a pressure control unit 37 to adapt the pressure provided at the mask,
- an Infrared bi-directional data communication system following the IrDa standard 28 used to allow wireless communications with the apparatus,
- a power supply manager 29,
- a motor driving system 36 which comprises a micro controller 26 and a power stage 27 connected to the centrifugal blower 33 by three wires 32.

The computerized board 19 is provided with energy by a battery pack 30 or an external DC power supply 31.

These different elements are connected each other as represented in figure 1 by the links with a single arrow on one or two extremities of a link. This can be an electrical or data's connection.

The motor driving system 36 is a mean for optimally using the efficiency of the electric motor of the air blower.

The adaptation of the air flow to a patient requires efficient motors. This is why sensors were used to know the position of the rotor, in order to change the rotor when at its optimal position. But as explained above, the sensors of prior art have several downsides.

The apparatus according to present invention has the capacity to precisely determine the rotor's optimal position, so that the efficiency of the electro motor enables the blower to have fast accelerations and decelerations within one patient's breath.

The solution provided by the invention is that instead of directly detecting the position of the rotor by the mean of sensors, it is detected by sensing the back electro motor force (BEMF) generated by the motor. This BEMF is detected on one branch of the stator which has three sectors.

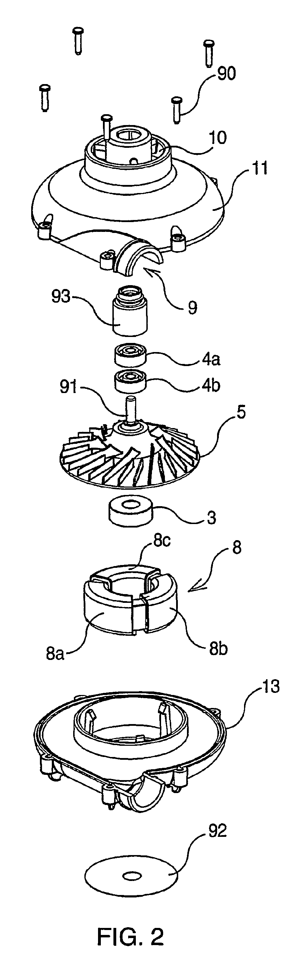

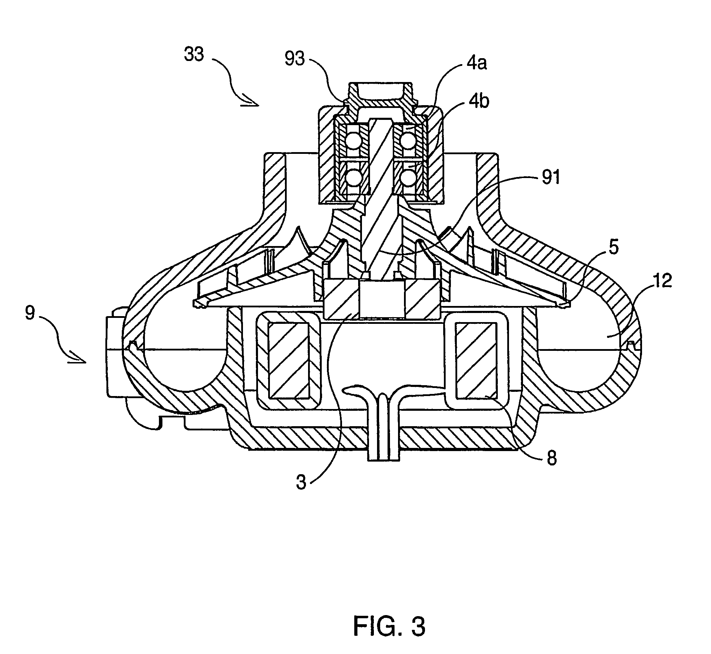

The air blower as represented in figure 2 and 3 is composed of:

- a top cover 11 which comprises an air inlet hole 10,
- an impeller/rotor 2 subsystem including two high speed bearings 4a and 4b, a rotor 3 which is permanent magnet with two poles and a twenty seven blades impeller 5, of for example 47mm diameter,
- a bottom cover 13,
- a three stator 8 with three sectors 8a, 8b and 8c.

Figure 2 represents also the screws 90 to fix the bottom and top covers together. Also is represented a sticker 92, which is added in the embodiment of blower represented to avoid air leakage.

When assembled the stator 8 and the rotor 3 composed the electric motor which rotates the impeller 5 which is attached to the rotor. The impeller rotation attracts air trough the air inlet 10 and compresses it in the chamber 12. The air is blown under pressure through the air outlet 9. The tube to patient's mask is connected to the air outlet 11.

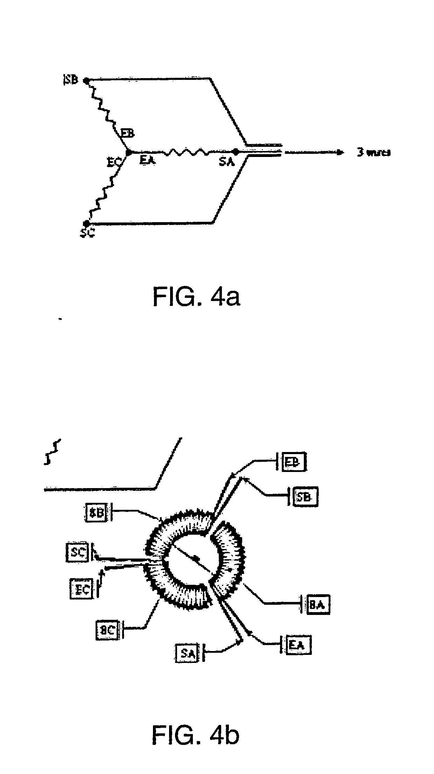

In a preferred implementation, represented in figure 4b, the stator 8 is a toroidal stator composed of three sectors (8a, 8b and 8c) which are coils. The two extremities S or E of each coil are grouped and join to a wire. As it is represented in figure 4a, this enables the stator to require only three wires to be connected.

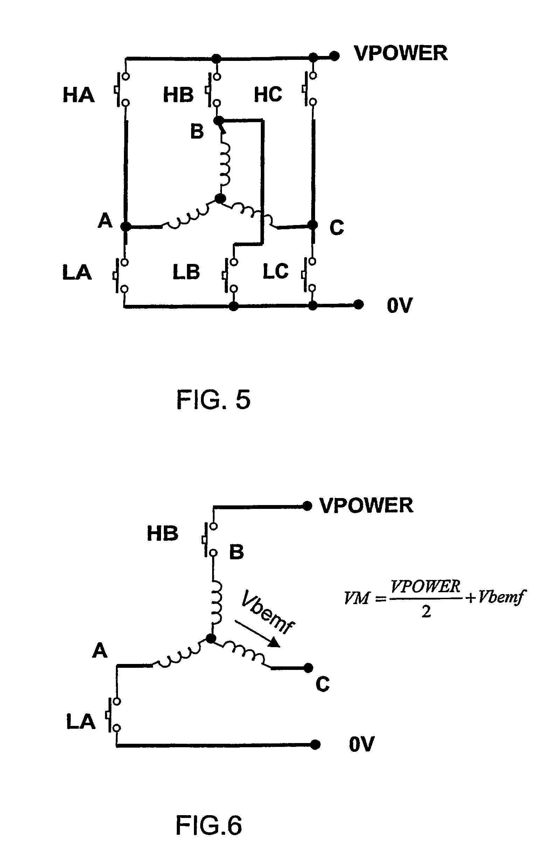

The figure 5 represents schematically the way the stator 8 with three sectors is connected to the power stage 27 which provides the motor with energy. Each of the three stator sectors 8a, 8b and 8c, is connected to respectively A, B or C. The switches HA, HB and HC (switches H) connect the positive plot of the power supply to each respective branch of the stator A, B and C. Each of these branches corresponds to one stator sector 8a, 8b and 8c. In the same way the switches LA, LB and LC (switches L) connect to the negative plot (0 V) of the power supply to the respective branches A, B and C of the stator. The motor functions by rotation steps. In each of these different steps, different phases are applied to each point A, B, C in order to rotate the rotor. Figure 6 represents one of these steps. In this figure, switches LA and HB are switched on applying the tension VPOWER delivered by the power stage 27 between branch B and A. The switches connected to C are switched off, thus enabling to measure the tension VM between C and the negative plot. In these conditions, this voltage obeys to the formula :

$$VM= (VPOWER/2) + Vbemf$$

wherein Vbemf represents the value of the BEMF.

This enables the micro controller 26 to measure the BEMF. The best instant to change the phase is when the rotor 3 arrives in the optimal position, which is when the rotor magnetic field and the magnetic field between A and B are in a situation of opposite polarity. Before the rotor reaches this position, the BEMF will be positive ; if the rotor pass this position, the BEMF will be negative. At the instant the rotor is at the optimal position the value of the BEMF equals zero. Thus, when the zero value is detected on C the second micro controller 26 sends orders to configure the switches positions to change the configuration of the stator branches polarity. Each change causes the rotor rotation.

This system enables the motor to be very efficient and thus enables high speed rotation and quick accelerations. By avoiding the use of sensors depending on their position, the apparatus according to present invention enables to rotate easily at a high speed. This high speed rotation enables to reduce the size of the impeller while providing the same pressure. The impeller has consequently a lower inertia this enables to further increase the acceleration of the motor.

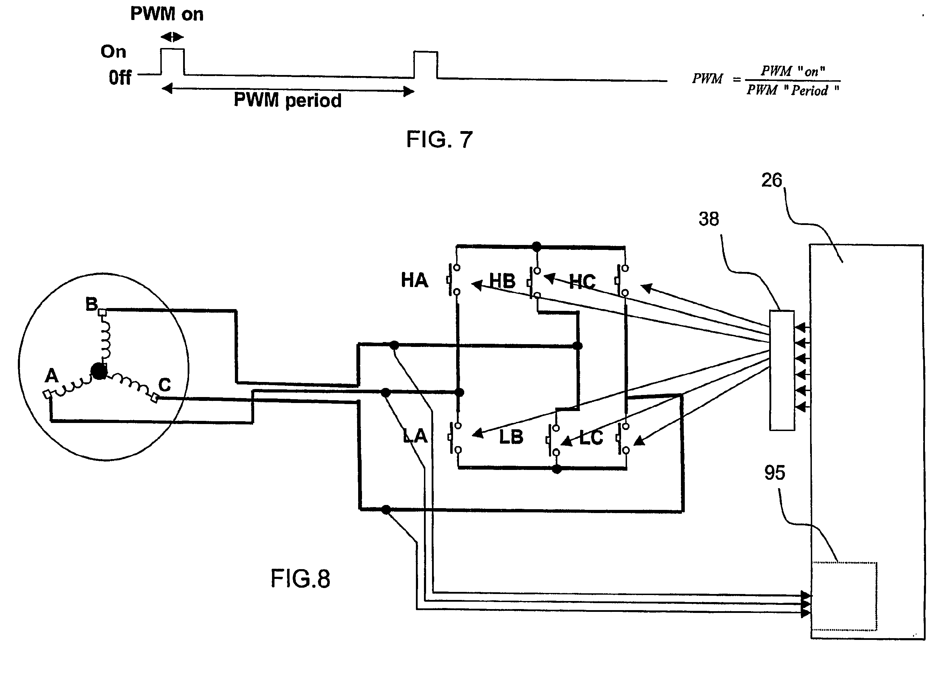

But in breath support it is also very important to provide good decelerations. When the tension is applied on one sector of the stator 8 the corresponding switch H is on. The tension is preferentially applied with pulses, such a tension is called a Pulse Width Modulation or PWM. This means that during a first duration of time the switch H is on, and that during a first duration of time the switch H is off, these two steps consisting in what is called the PWM period. Then H is switched on again and a second PWM period begins. The figure 7 represents the PWM value related to time evolution. Because of the short time of the PWM period (typically between 50 and 100 $\mu$s), the tension in the system PWM is given by the formula:

$$PWM = (PWM"on" / PWM"period") X VPOWER$$

VPOWER being the tension the driving unit is provided with

For example, during the PWM period when HA is off, if LA is also open, the system functions correctly at high speeds and with fast accelerations but has no possibility of fast decelerations. To decelerate it is required to decrease the PWM value, which is done by shortening the period when the tension is applied (PWM"on" with the switch HA on). Thus the tension applied will decrease but the BEMF will not decrease immediately. None of the switches settings is allowing the BEMF to be drawn to either rail of the power supply. This means that when PWM reaches the zero value and when the switch HA is on, it will be similar to have a left opened free wheeling motor which only breaks by mechanical bindings (in bearings for example) and according to the airflow load (airflow driven out). To implement the system and provide fast decelerations, a solution is to switch the LA switch on during the period when HA is a off. Thus the current generated by the motor will be sent via LA to the system. In case of PWM decrease the motor will provide a motor braking which enables a fast deceleration.

As an implementation of the invention is to switch on one of the L switches during the PWM period and to switch the switch H of the same branch off at the same time, a limitation appears if having PWM on both sides of a branch during the transition time, physical switches such as MOSFET transistors are having delays to change their state from ON to OFF or OFF to ON. This means that the transitional condition where the two switches, L and H, of the same branch are on, can occur resulting on a direct short cut of the power supply. The implementation thus requires to generate a dead time period between the H switch switching OFF and the L switch of same branch switching ON. This can be generated by a software and a micro processor comprised in the driving unit.

The figures 8 represents how the stator and the switches are connected to the micro controller 26. As represented in figure 8, the stator is connected to the switches set by only three wires, each stator sectors being connected on A, B and C as described above. Each point A, B, C is connected to the micro controller 26 which enables to measure the BEMF as described above. The micro controller 26 send the numerical orders to an adapter 38, which converts these orders into a position of the corresponding switch, so that the driving unit is enabled through a micro controller's software to switch each switch off or on.

The following table 1, shows the functioning of the controller 26 during one rotating sequence (one complete rotation of the rotor).

Tableau 1:

| STEP | 1 | 2 | 3 | 4 | 5 | 6 |
|---|---|---|---|---|---|---|
| Cxout | | | | | | |
| 0(HA) | PWM | PWM | 0 | 0 | 0 | 0 |
| 1(LA) | !PWM | !PWM | 0 | 1 | 1 | 0 |
| 2(HB) | 0 | 0 | PWM | PWM | 0 | 0 |
| 3(LB) | 1 | 0 | !PWM | !PWM | 0 | 1 |
| 4(HC | 0 | 0 | 0 | 0 | PWM | PWM |
| 5(LC) | 0 | 1 | 1 | 0 | !PWM | !PWM |
| | | | | | | |
| BEMF ON: | C | B | A | C | B | A |

In this preferential mode as the stator has three sectors and as the rotor is a dipole magnet, the sequence comprises six steps corresponding to the six different ways of applying the polarity to the stator branches in order to rotate the motor in one sense. The state of each switch is represented in respect of each step. For example in step 1, HA is switched on during the PWM on time (and off during the rest of the PWM period), LA is switched on during the rest of the PWM period (noted ! PWM). HB, HC, and LC are switched off and LB is switched on. During this step, A has got the positive polarity and B the negative polarity. The BEMF is measured on C. This measure is sent to the micro controller 26. When the BEMF reaches the 0 value, then the command are operated to gave the instructions to switch the switches in the configuration of step 2. In step 2 the positive polarity stays on A, the negative polarity is on C, enabling a 60 degrees rotation of the rotor. The BEMF is measured on B, and so on for each step.

Thus, the efficiency of the motor driving system 36 allows very fast and accurate accelerations and decelerations of the motor. As the rotation of the motor rotates the impeller and as the impeller rotation speed is a function of the pressure provided by the blower, this efficiency enables fast rise and fall of pressure. Typically under normal airflow load conditions, the blower will rise from 1 hPa (hecto pascals) to 30 hPa in less than 200 milliseconds under the same conditions, the blower will drop from 30hPa to 1 hPa in the same duration. The rise or fall can be of more than 10 hPa in 30 to 60 milliseconds.

For the intended use of the apparatus, those performances are giving to the device enough latitudes to accomplish an enhanced pressure regulation at the patient mask and a multi level of pressures within breaths without the requirement of additional valves.

This provides the maximum efficiency of motor rotation, and enables it to rotate at high speed (more than 50000 rounds per minute) and to have fast acceleration or decelerations, that is to say at least a

In the preferred embodiment the stator has only three sectors. Even if it would be more complicated, it could also be used stators with more sectors. For example a six sectors stator could be used with a rotor being a magnet with four poles.

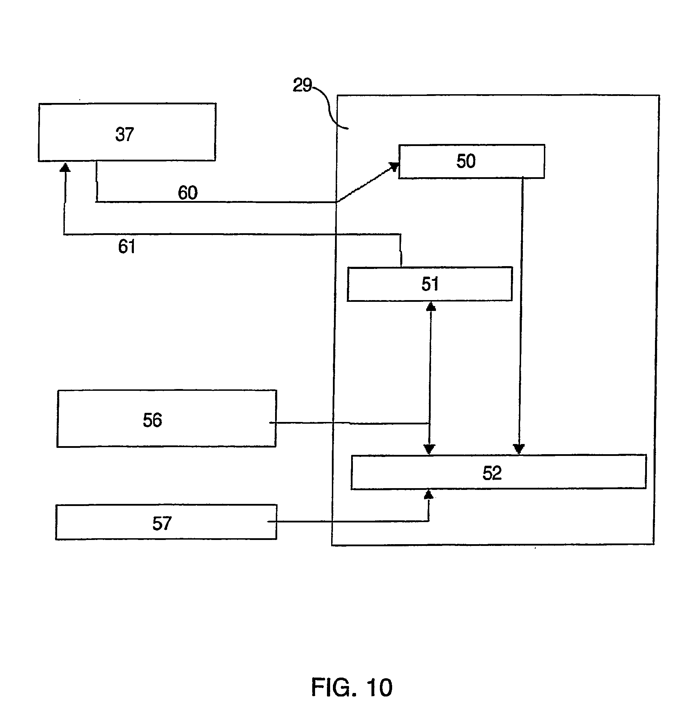

In a preferred embodiment the apparatus has a power supply manager 29 which is connected to a battery pack 30 or to the power supply sector via an external plug 31. The power supply manager comprises a communication module which enables to avoid expensive connectors and cables. This module is used to transmit data trough the power source wires. As represented in figure 10, the pressure control unit 37 transmit a binary data flow 60 to a Frequency Shift Keying (FSK) modulator 50 which transforms these binary data in a modulation of the tension frequency of the tension applied on the voltage controlled current source 52. The external power supply is connected on the voltage controlled current source 52 with its 0V plot 57 and its positive plot 56, so that the voltage controlled current source 52 transmit voltage frequency and possibly the modulation corresponding to the data. In the same way when a modulated voltage is applied by other modules or sensors on the positive plot 56, a FSK demodulator will convert the voltage frequency modulation into binary data 61 and transmit them to the pressure control unit 37. This voltage is transmitted by the positive plot 56, so that each sensor or module connected to the power source is able to receive or transmit information.

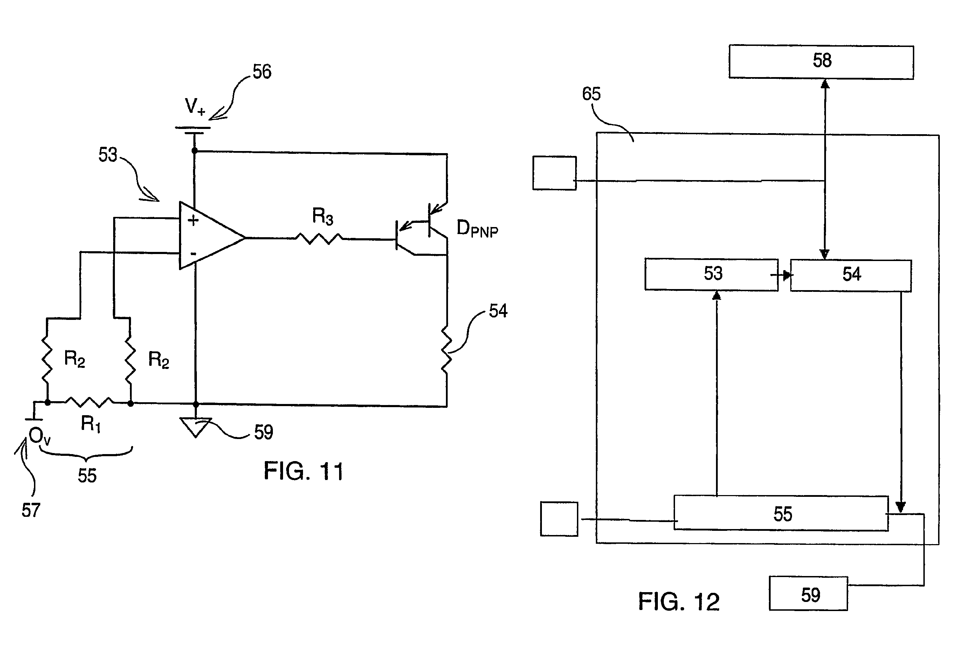

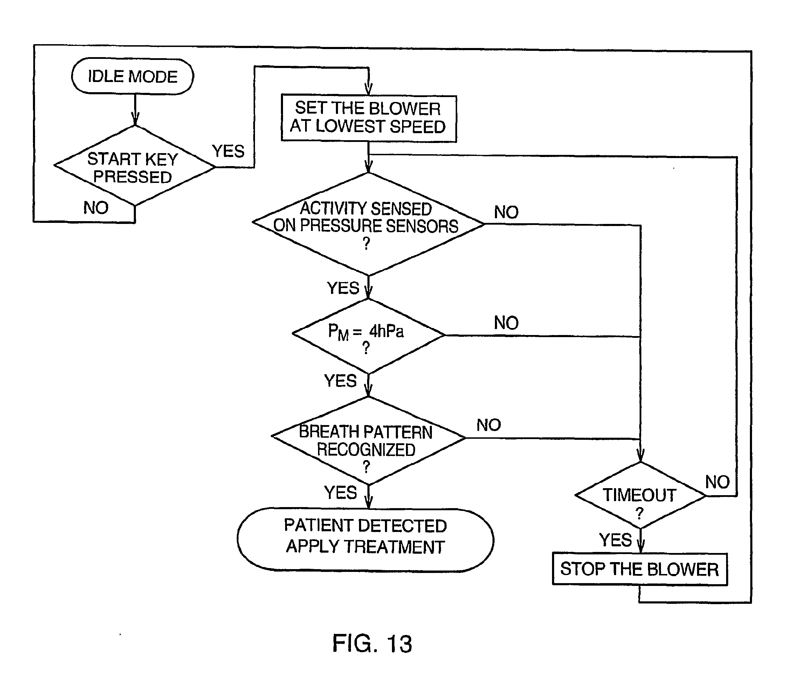

In a further embodiment, the power supply manager 29 can comprise a load control module. The high speed centrifugal blower 33 has a motor 8 and 3 and regardless the motor technology, when a slowdown of the motor is required it induces a negative current from Ground to positive plot of the power supply, because the motor in that condition acts as a generator. If the motor decelerates, as for example described above with the implementation for enabling fast decelerations, this negative current is generated back into the system and will be sent into the power supply. When the power of the power supply only delivers energy to this apparatus and as a power close to the apparatus consumption power, serious damages can occur. The load control module aim is to prevent those downsides. As represented in figure 12 and 13, this load control module 65 comprises a current sensor 55, preferentially a 0.1 ohm resistor $R_1$ connected to the external power supply and to a comparator 53, the load control module 65 further comprising a load resistor 54 which can be connected by switches to the positive power supply 58 and to the ground 59. The comparator 53 is connected to the load resistor 54. The current sensor 55 provides the comparator 53 with the value of the current, so that when this value is negative the comparator 53 switches the load resistor 54 on between the positive power supply 58 and the ground 59 by the way for example of a Darlington PNP, or $D_{PNP}$, thus allowing the excess of negative current to be drawn in the load resistance 54 thus allowing the dissipation of the energy generated by thermal effect. Such device is particularly useful in the present invention but is not necessary and could be applied in other breath assisting apparatus like CPAP. The electric scheme of this implementation is represented in figure 11.

Preferentially, the apparatus according to the present invention further comprises bearings 4a and 4b and a bearing holder 93. The impeller 5, said rotor 3 and the impeller's shaft 91 are fixed together, said bearings being fixed to said axis and being hold inside the bearing holder. Thus the impeller, the rotor and the bearings inner ring rotate together with the shaft. The bearings outer ring being hold in the bearing holder, which is fixed in the apparatus. Instead of being centered inside the stator 8, the rotor is shifted outside the stator 8, preferentially at an equal distance of each of the three stator sectors 8a, 8b and 8c, so that the stator also generates on the rotor 3 an axial force oriented along the impeller's shaft 91, thus generating a preload on the bearings 4a and 4b. On bearings, a preload is required to operate smoothly and quietly. This further increases the motor efficiency. An other advantage is that it avoids the use of springs to generate the preload on the bearings.

The BEMF detection for controlling the blower motor allows high speed of impeller rotation. One advantage is that the noise gets in higher frequencies. This enables to insulate the blower with a smaller box 34 and enables to have less noise. By preferentially increasing the number of blades the apparatus according the present invention further moves the noise of the apparatus at higher frequencies. This number of blades is comprised between 14 and 45, preferentially 27. It is preferred to have an impair number of blades.

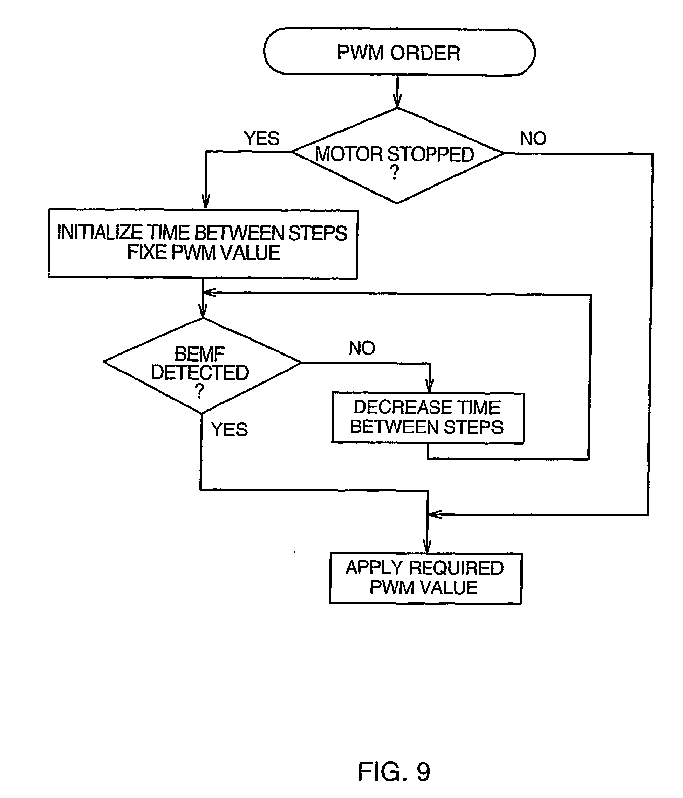

An implementation of the present invention is to force the rotor 3 to rotate at a speed high enough to enable the BEMF to be detected. To this aim, when receiving a PWM value the micro controller 26 will follow the process as represented on figure 9. If the motor is not stopped then a request to apply the tension PWM value to the stator 8 is sent. If the motor is stopped, the micro controller 26 initializes the time between the rotor's rotation steps, these steps consisting in changing the phases of the stator branches. In that case, the micro controller 26 also fixes the PWM value. This means that until the BEMF is detected, the changes of phases depend on time and not on a BEMF detection. The micro controller 26 keeps on decreasing the time between the changing phases steps, until the BEMF is detected. When the BEMF is detected, the tension PWM value corresponding to the pressure required is applied. The apparatus function at this time with BEMF detection.

Another advantage of a BEMF detection is that the fastest the motor will turn, the more precise the BEMF detection will be resulting on an increase of efficiency.

An other embodiment of the invention is to obtain the stator 8 by using a strip wound cores with a high grade thin silicon steel. It is known that to improve stator efficiency the number of laminations have to be high to reduce core losses. The classical solution in motor design is to slice the stator in a high number of laminations with a thin isolation. According to the present implementation another method is used to obtain the stator: using a strip wound technique allowing to go to 0.05 mm thickness of the steel. This technique used in toroidal transformer has been here adapted to the apparatus

Another advantage of the invention is that, the high rotation speed and quick rise time and fall time also enables to use tubes of a smaller size. Historically CPAP and respiratory assistance devices are using 1.8m long, 22mm outside diameter tubing, because the pressure drop on those tubes are negligible (0.7hPA for 1l/s airflow). Pressure drops are happening during inspiration and, during expiration, pressure is increasing. In that case it is important that the pressure of the provided air does not change too much ; this is the reason why 22mm diameter tubes are used. The downsides of those tubing's are size and weight. It can be interesting to use smaller tubes given the reduced size of the apparatus. The downside of a reduced diameter tubing is the fact that significant pressure drops are occurring. Given the light weight of the impeller/rotor 2 combined with the efficiency of the motor driving system 36 and the load control of the power supply manager 29 allowing very fast and accurate accelerations/ decelerations, the apparatus according to the present invention is able to compensate those pressure drops thus allowing the use of smaller tubing's (i.e.15mm outside diameters).

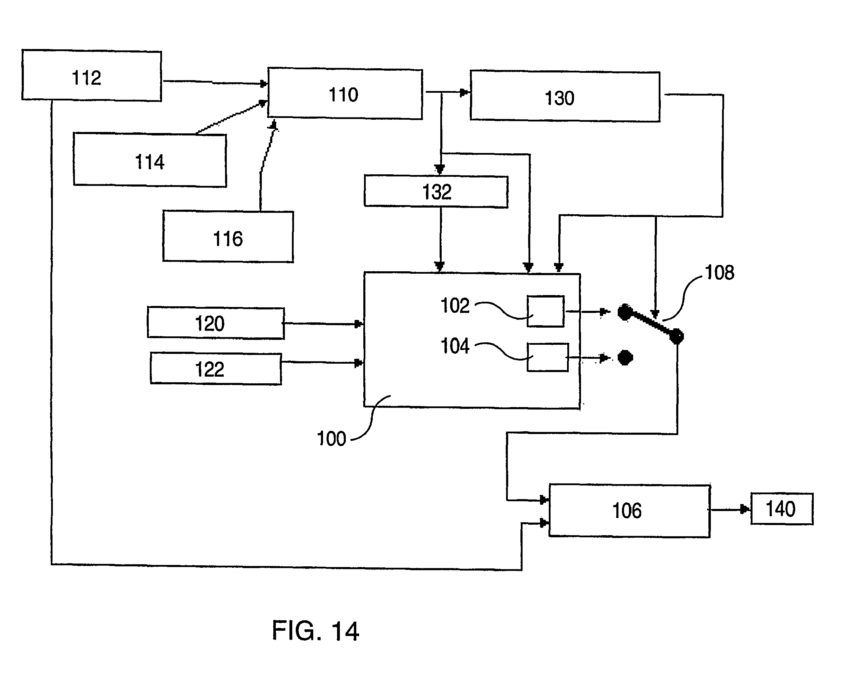

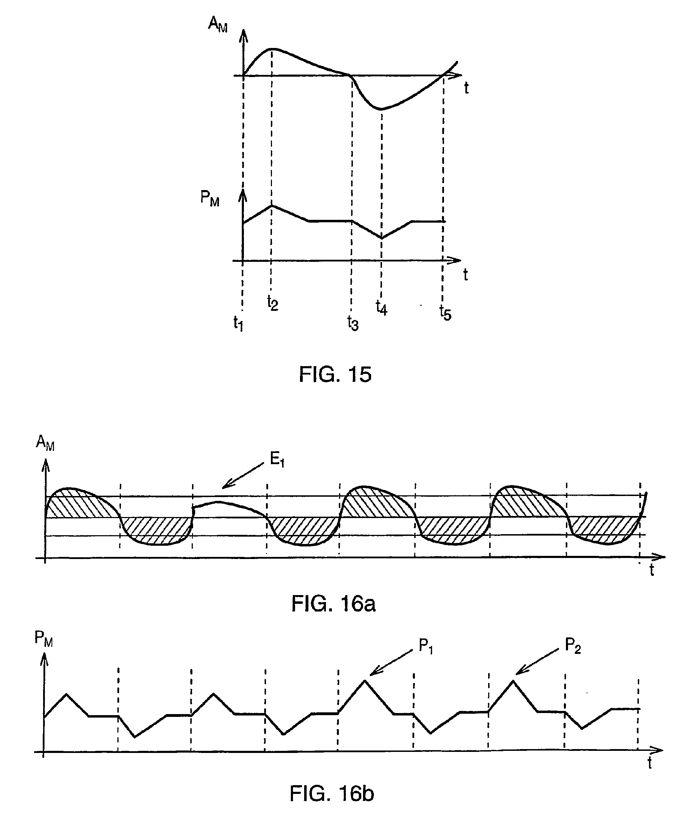

In a preferred embodiment of the present invention, the apparatus comprises means for measuring the patient's breathing parameters at the patients mask. These means are preferentially means that enables to determine the airflow. The apparatus allowing fast accelerations and decelerations allows to modulate the pressure to the patient in respect to the illness to treat. Based on the high speed and efficiency of the centrifugal blower, the apparatus can accommodate almost any kinds of pressure patterns to any kinds of breaths but only a few among those are useful in the respiratory treatments. The apparatus has thus the ability to differentiate the two basic states of the respiration: inspiration and expiration. Two of consecutive basic states constitute one breathing step. The sensors provided in the apparatus enables the pressure control unit 37 to determinate the pressure required for the patient and to send the corresponding Pulse Width Modulation PWM value to the micro controller 26. Preferentially the pressure control unit comprises an estimation module 100 or program which determine the pressure to apply, and comprises a control loop which converts this value of pressure into the tension PWM to apply to the blower. The determination by the pressure control 37 of the tension PWM to apply is represented in figure 14. The outputs of the estimation module 100 are the value of the inspiration pressure PI which is the pressure maintained at the patient's mask during the inspiration, and the value of the expiration pressure PE which is the pressure maintained at the patient's mask during the expiration. The patient's breathing parameters, and preferentially the airflow parameters, enables the computation of the inspiration and expiration, this latest computation enables the estimation module 100 to determinate, which step of the patient's breathing is occurring. A breath estimation module 132 is qualifying a breath in shape, energy (volume) and frequency. The clinician or a qualified user enters parameters or shapes of the delivered pressures for the expiration phase and the inspiration phase. The clinician entered also parameters 120 defining how the estimation module 100 is going to react following events detected in the breath estimation module. It is well known that a feedback of the patient with his treatment is helping compliance, thus the patient can have an access to a parameter 122 ranging from minimum to maximum that is qualified to be "comfort vs efficiency". This patient setting 122 is having the weight that the clinician is giving to it, from pure placebo effect to some level of effects. Basically the patient's settings are applied in the normal breath situation or/and have a limited action on the pressure regulation. It is also possible that the airflow is an input to the estimation module 100. Thus, with the data inputs concerning the breath estimation (and clinical symptoms or event associated with), the inspiration/expiration computation and the clinical settings, and possibly the airflow computation and patient settings, the pressure control unit 37 by the estimation module 100 is able to determinate the pressures required PI and PE. Those two values can be addressed to two different outputs where a switch is able, relative to the inspiration/expiration computation, to connect to the required output regarding if the patient is breathing in or out. The pressure control unit 37 comprises a pressure control loop 106 which, by comparing the pressure measured in the mask and the value of pressure required PI or PE, is able to adjust the tension PWM in order to obtain the correct pressure in the mask. The figure 15 represents one pattern of the pressure of treatment provided according to the airflow due to the patient breathing. In this example, the clinical technician has set a special modulation of pressures PI and PE during respectively the inspiration and the expiration; after a while as no special event occurs the values of the two pressures are changed. The estimation module 100 can preferentially use a clock time 25 to time mark the breathing parameters.

The apparatus has a two steps, strong recognition, process in order to prevent false start of the apparatus when the mask is not on the patient's face and to prevent starting a new treatment session. When the apparatus is started by the patient by using the keyboard, for example and as represented in figure 13 by using the start key, the blower 33 is kept turning at a very low speed, waiting for some activity on the mask pressure sensors 23. When an activity is detected, the apparatus is instantaneously trying to bring the pressure at the apparatus outlet 9 at a minimum starting pressure $P_0$ of 4 hPa. This will guarantee that no $CO_2$ rebreathing will occur. When this pressure is reached the estimation module 100 tries to identify at least one breath to start the treatment process according to the settings. When the mask is not applied against something, like a hand or the patient's face, no activity is detected. Then if a maximum time has been spent (the timeout is reached) since the start key is actuated, without detecting any activity, the blower is stopped. On the contrary, the apparatus keeps on waiting for an activity on the pressure sensors. When the mask is not applied correctly the pressure can not reach 4 hPa. Then if the timeout is reached the blower is stopped, on the contrary the apparatus tries again to detect some activity on the pressure sensors. When the mask is not applied on the patient's face no breath pattern is recognized. Then the blower is stopped if the timeout is reached. On the contrary the estimation module 100 waits to detect some activity on the pressure sensors. The timeout checking prevents the blower keeping turning on if the patients does not start the treatment and forget to start the blower.

Preferentially, the data of the pressures PM and PB which are sensed at the extremities of the tube and the data of the tube coefficient $K_T$ enable the airflow computation. One formula that can be use to compute the airflow from these values is :

$$\texttt{Airflow}^2 = (\texttt{PM-PB})/\texttt{K}_\texttt{T}$$

The following examples demonstrate the way the estimation module 100 modulates the pressure value PM to apply to the patient's mask.

Example 1: Pressure Pm at patient's mask within a breath

As represented in the figure 15 the pressure provided is not constant but is a function of time.

At time t1 the patient starts breathing in. Time t2 is defined when the absolute value of airflow starts to decrease within the inspiration phase or shows a fixed delay after t1. As the estimation module is calculating the derivative of the airflow function, it is able do determine this time for example when the derivative reaches zero. Time t3 is the start of expiration. Time t4 is defined when the absolute value of airflow starts to decrease within the expiration phase or shows a fixed delay after t3. Time t5 is the end of the breath step and the beginning of a next breath step.

In order to reduce the patient's lung effort the estimator increases the value of pressure PM from time t1 to t2. After time t2 as the absolute value of airflow is decreasing the estimation module decreases the pressure value. In the same way, to reduce the patient's lung effort the estimator decreases the value of pressure PM from time t3 to t4. After time t4 as the absolute value of airflow is decreasing, the estimation module increases the pressure value.

The value of PM given by the estimation module and thus the pressure applied at the mask is a function of time.

Figure 15 represents one example of the PM function pattern on one breath step. This function is given by the following formula :

$$\text{if } t1 < t \leq t2: \qquad P_M = P_T + (t - t1) \times S_{LH}$$

$$\text{if } t2 < t \leq t3 : \qquad PM = MAX(P_T + (t2 - t1) \times K_{RH} - (t - t2) \times SLH, AVP)$$

wherein the MAX function returns the greatest of its two members, each member being separated in the parenthesis by a coma.

$$\text{if } t3 < t \leq t4 : \qquad PM = P_T + (t - t3) \times SLH$$

$$\text{if } t4 < t \leq t5: \qquad PM = MIN(AVP - (t4 - t3) \times SLH + (t - t4) * SLH, AVP)$$

wherein the MIN function returns the smallest of its two members, each member being separated in the parenthesis by a coma.

According to the figure 15 and these equations, the increases and decreases are linear, the absolute value of the slope coefficient $S_p$ being a constant set by the patient or the clinician. During one inspiration the pressure value Pm is always superior or equal to treatment pressure PT. In figure ? the pressure is constant just before t3 (as the first member of the MAX function is inferior to PT. During one inspiration the pressure value Pm is always inferior or equal to treatment pressure PT. On one breath step the mean of the average pressure value PM is equal to the treatment pressure PT.

Clinician could also define bounds wherein the estimation module would limit the SP value when SP is set by the patient.

This kind of operation is allowed with the apparatus according to the present invention, because it has the ability to precisely and quickly regulates the pressure.

Example 2 : Variations of the average pressure of treatment as a function time according to detected events

In a preferential embodiment the average pressure of treatment within one breath, that is to say within one breathing step, can be modulated according to the events occurring, such as snoring or apneas or abnormal breathing patterns.

The apparatus will normally (no events detected) try to reduce the average pressure of treatment value, thus enhancing the patient comfort while breathing against the apparatus. The clinician set a minimum average pressure of treatment Ptmin and a coefficient $N_{OEK}$ expressed in hPa/s .

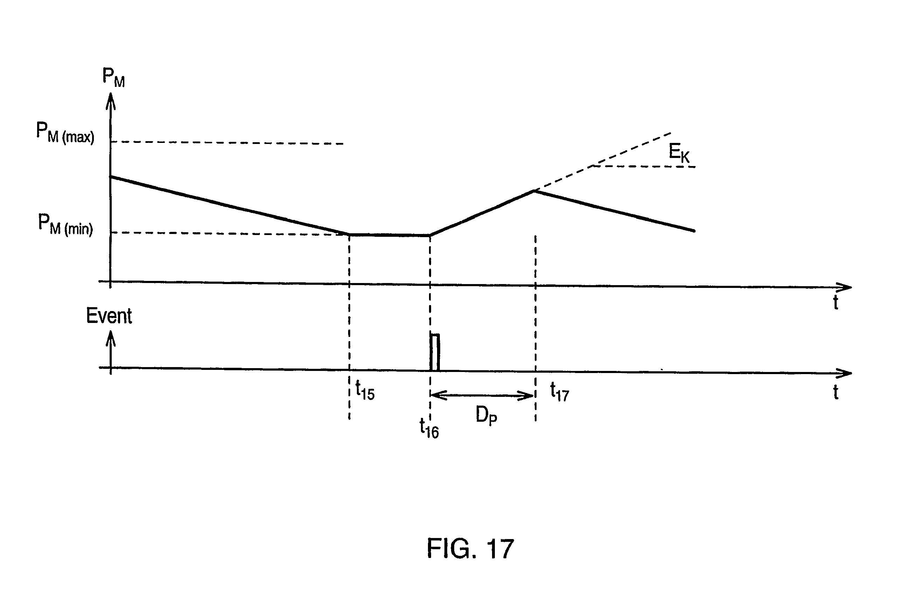

In the following example the terms AVP, are the average pressure of the pressure to patient's mask on one breath step. On figure 17, this average pressure as been noted PM, as the measurement is made on the first pressure sensor 6. As represented on figure 17, when no events are detected the average pressure of treatment value will follow

the equation:

$$AVP(t) = MAX(AVP(t - \varepsilon) - (NOEK \times \varepsilon), AVP\min)$$

$\varepsilon$ being the sampling time, which is the duration wherein the calculation is made, this sampling time corresponding to one breath step, the MAX function is returning the greatest value of its two members.

The average pressure value Pm thus decreases linearly until it reaches the minimum set by the clinician and stays constant until an event occurs.

When an event is detected, a 3 steps process is initiated :

- Step 1: the estimation module, looking in the clinical settings is defining if the event has to affect the average value of pressure PT.
- Step 2 : If so the estimation module looking in clinical settings, will define a persistence delay Dp.
  In the example of figure 17 a snore is detected, the persistence delay could be set to 2 minutes.
- Step 3 : A **Ek** parameter in hPa/s is extracted from clinical settings and balanced with an eventual ongoing event. The estimation module will determine the Ek corresponding to the event which occurs or has occurred and linearly increase the average pressure of treatment with Ek as slope coefficient. AVP will then follow the equation:

$$AVP(t) = MIN(AVP(t - \varepsilon) + (EK \times \varepsilon), \overline{AVP}\max)$$

$\varepsilon$ being the sampling time, the MIN function is returning the smallest value of it's two members.

During the persistence delay even if no event occurs the estimation module will keep on increasing the average pressure of treatment PT.

Example 3 : Variations of the slope SLH of the value of pressure PM applied to the patients mask within a breath.

The slope coefficient of the pressure applied function of time within a breath can also be modulated according to time. In a preferential mode this coefficient remains constant on each single breath step (one inspiration and one expiration), but can be changed from one step to another.

The apparatus will try to reduce the slope coefficient SP, thus enhancing the patient comfort while breathing against the apparatus. The clinician set a minimum the slope coefficient SP, and a coefficient $N_{SP}$ expressed in hPa/s.

The events are detected the slope coefficient SP value will follow the equation :

$$SLH(t) = MAX(SLH(t - \varepsilon) - (NOES \times \varepsilon), SLH\min)$$

$\varepsilon$ being the sampling time, the MAX function is returning the greatest value of its two members.

The shape of the corresponding curve is like the one for average pressure in figure 17.

When an event is detected, a 3 steps process is initiated :

- Step 1: the apparatus, looking in the clinical settings is defining if the event has to affect SP
- Step 2 : If so the apparatus looking in clinical settings, will define a persistence delay further named **DS**.
  If, as represented on figure 18 a snore is detected, the persistence delay could be set to 2 minutes.
- Step 3 : A E**S** parameter in hPa/s$^2$ is extracted from clinical settings and balanced with an eventual ongoing event. The estimation module will determine the Ek corresponding to the event which occurs or has occurred and linearly increase the average pressure of treatment with ES as slope coefficient. SP will then follow the equation :
- 

$$SLH(t) = MIN(SLH(t - \varepsilon) + (ES \times \varepsilon), SLH\max)$$

$\varepsilon$ being the sampling time, wherein the calculation is performed, preferentially with a 200 Hz frequency the MIN function is returning the smallest value of it's two members.

During the persistence delay even if no event occurs the estimation module will keep on increasing the slope SP.

Example 4 : d1, d2 auto adjustment

On the figures ? d1 and d2 have two different behaviors, according to t2 and t4 definitions. **t2** is defined when the absolute value of airflow starts to decrease within the inspiration phase or shows a fixed delay after t1. **t4** is defined when the absolute value of airflow starts to decrease within the expiration phase or shows a fixed delay after t3.

If t2 and t4 are defined according to the airflow waveform then no auto-adjustment is occurring. If not, following the same rules than SLH or AVP, the d1 and d2 can also be affected by the events. In the case when d1 and d2 are not locked by the breath waveform following the same process, d1 and d2 can also be adjusted according to events in the case.

**Claims**

1. An apparatus (1) to assist a patient respiration by delivering air to this patient trough a mask, comprising an air blower (33) wherein the impeller (5) is rotated by an electro motor comprising a rotor (3) and a stator (8), said stator having at least three sectors (8a, 8b and 8c), the rotation of the rotor being enabled by changes of the polarity of the sectors, each sectors polarity configuration constituting one step of the rotor's rotation, said apparatus comprising a driving unit (36) controlling changes of the sectors polarity configuration such that the electro motor enables the blower to achieve fast accelerations and decelerations within one patient's breath step, said breath step consisting of one inspiration and one expiration ; **characterized in that** said driving unit comprises means to sense the back electro motor force generated by the electro motor and that said driving unit is configured to change the sectors polarity configuration when the back electro motor force reaches the zero value.

2. The apparatus (1) according to claim 1, wherein said stator (8) has at least three sectors (8a, 8b and 8c), each of said sectors being connected to one switch (HA, HB, or HC) connected to the positive plot of the power supply and each of said sectors being connected to one switch (LA, LB or LC) connected to the negative plot of the power supply, in order that one of the rotor's rotation step is obtained when said driving unit (36) applies the tension to the stator by connecting the first sector (8a) to said positive plot, connecting the second sector (8b) to the negative plot and setting the third sector (8c) not connected to a power supply plot, thus enabling to measure the back electro motor force of the motor between the third sector and the negative plot, said driving unit changing the sectors polarity configuration when the back.electro motor force reaches the zero value.

3. The apparatus (1) according to claim 2, wherein said tension applied is a Pulse Width Modulation, the driving unit connecting one of said stator sectors (8a, 8b or 8c) to the positive plot during a first duration of time and then, and then, during a second period, disconnecting the same sector from the positive plot and connecting it to the negative plot, so that in case of deceleration of the motor the generated current is sent to the negative plot, thus providing a fast deceleration of the impeller (5).

4. The apparatus (1) according to claim 2 or 3, wherein said stator (8) is a three sectors stator and said rotor is a dipole magnet, said stator thus having six sectors polarity configuration so that the rotor performs one 360° rotation in six rotation steps.

5. The apparatus according to any one of claims the previous claims, wherein said.stator (8) is a toroïdal stator and wherein each of said sectors (8a, 8b or 8c) are coils connected with only one wire.

6. The apparatus according to any one of the previous claims, wherein when the blower (33) is functioning and no back electro motor force is measured, said driving unit (36) fixes the tension applied and changes the sectors (8a, 8b and 8c) polarity configuration after a given time, said driving unit decreasing this given time every step until a back electro motor is detected and then applying the required tension and changing the sectors polarity configuration according to the back electro motor value.

7. The apparatus according to any one of the previous claims, further comprising bearings (4a and 4b) and a bearing holder (93), and wherein said impeller (5), said rotor (3) and the shaft (91) of said impeller are fixed together, said inner ring of the bearings being fixed to said shaft and the outer rings of said bearings being hold by the bearing holder, which is fixed in the apparatus, and said rotor being shifted outside the stator, preferentially at an equal distance of each of the three stator sectors (8a, 8b and 8c), so that the stator also generates on the rotor an axial force oriented along said shaft, thus generating a preload on the bearings.

8. The apparatus according to any one of the previous claims, further comprising a power supply manager (29) connected to the power supply source (30 or 31), said power supply manager comprising a current sensor (55), a comparator (53), a load resistor (54) and a mean to switch on the load resistor between the positive power supply and the ground when the current measured by said current sensor is negative, in order to dissipate this current in said load resistor by thermal effect.

9. The apparatus according to any of the precedent claims, being designed to be connected to a tube of a diameter less than 22 millimeters, a first extremity of the tube being connected to the air outlet of the blower (33) and a second extremity of the tube being connected to a mask in which the patient breaths.

10. The apparatus according to any of the precedent claims further comprising :

    - at least one means (110) for detecting the patient's breathing parameters,
    - a pressure control unit (37) to adjust the pressure delivered by said blower at the level of said mask, and comprising an estimation module (100) connected to the means for detecting the patient's breathing parameters, in order that the estimation module is able to determine when the patient is inspiring or expiring and in response the pressure to apply to the patient's mask, during inspiration and during expiration.

11. The apparatus according to claim 10, wherein said at least one means detects the patient's airflow and send it to a breath estimator (132) which determines the airflow as a function of time and transmit this function to said estimation module (100) which will thus estimate the pressure to apply to patient's mask according to the airflow function, in order to decrease the effort of the patient's lung while maintaining, during one breath step, the average value of the pressure at the mask $P_M$ equal to the pressure of treatment, the estimation module (100) preferentially determining the pressure $P_M$ at the mask as a function of time.

12. The apparatus according to claim 10 or 11, wherein the control unit comprises a non volatile memory in which the clinician can enter clinical settings (120) comprising at least the treatment pressure and possibly the pressure to apply according to the patient's breathing parameters, said estimation module providing the pressure PM according to these clinical settings and to the patient's breathing parameters.

13. The apparatus according to claim 12, wherein the patient can enter patient settings (122) in said non volatile memory, said estimation module (100) providing the pressure according to these patient settings and to the patient's breathing parameters within bounds given by the clinician settings (120).

14. The apparatus according to any one of claim 10 to 13, in.which the estimation module (100) is able to determine that an event ($E_1$, $E_2$ or $E_3$) occurs in patient's breathing thus enabling said pressure control unit (37) to provide the blower (33) with the tension to apply to adjust the pressure at patient's mask.

15. The apparatus according to any one of claim 10 to 14, wherein said means (22 and 23) for detecting the patient's breathing parameters enable the pressure control unit (2) to compute the airflow at patient's mask, said estimation module (100) determining that an event ($E_1$, $E_2$ or $E_3$) is occurring with the airflow parameters or shape.

16. The apparatus according to claim 10 to 15, wherein said estimation module (100) has an inspiration output (102) where said estimation module set the mask pressure PM value during inspiration, and wherein said estimation module has an expiration out put (104) where said estimation module set the mask pressure PM value during expiration, said pressure control unit (37) comprising a switch (108) which is connected alternatively to the inspiration output or expiration output according to patient's breathing.

17. The apparatus according to claim 10 to 16, wherein the means for detecting the patient's breathing parameters comprise a pressure sensor (23) for sensing the pressure at said first tube extremity and one pressure sensor (22) for sensing the pressure at the extremity of the tube connected to the blower outlet, said airflow computation module being able to calculate the airflow from these pressures and from the tube airflow resistance coefficient KT.

18. The apparatus according to claim 10 to 17, wherein the apparatus further comprises a starting mean which, when actuated, orders the estimation module (100) to detect a breathing activity, said estimator module sending the instruction to stop the blower (33) if no activity is sensed after a given delay.

19. The apparatus according to any one of the previous claims, wherein a power supply manager (29) comprise a communication module (65) which transmits the data through the power source wires.

20. The apparatus according to any one of the previous claims, said communication module (65) comprises a Frequency Shift Keying (FSK) modulator (50) which transforms the binary data send by the apparatus sensors or elements in a modulation of the frequency of the tension applied on a voltage controlled current source (52), connected to the external power supply, so that the voltage controlled current source transmits the modulation corresponding to the data, a FSK demodulator converting the voltage frequency modulation into binary data (61) and transmits it to the elements, so that each sensor or module connected to the power source is able to receive or transmit information.

21. The apparatus according to any of the previous claims, further comprising a phonic insulation box (34) wherein the.blower (33) is placed, said impeller (5) having a size less than 60 mm and comprising between 15 and 45 blades, preferentially 27, so that the impeller rotates at a speed that generates a sound at a high frequency, enabling said box to insulate the patient from this noise.

22. The apparatus according any one of the previous claims, said apparatus being adapted for use for the treatment of breathing anomalies, such as snoring, apneas, or hypopneas.

23. The apparatus according to claim 5 to 22, wherein said stator (8) is obtained by a strip wound cores technique with a high grade thin silicon steel, of about 0.05 mm thickness.

**Patentansprüche**

1. Ein Gerät (1) zum Unterstützen der Atmung eines Patienten durch Liefern von Luft zu diesem Patienten durch eine Maske, das ein Druckluftgebläse (33) beinhaltet, wobei das Gebläserad (5) durch einen Elektromotor, der einen Rotor (3) und einen Stator (8) beinhaltet, gedreht wird, wobei der Stator mindestens drei Sektoren (8a, 8b und 8c) aufweist, wobei die Drehung des Rotors durch Änderungen der Polarität der Sektoren ermöglicht wird, wobei die Polaritätskonfiguration jedes Sektors einen Schritt der Drehung des Rotors festlegt, wobei das Gerät eine Antriebseinheit (36) beinhaltet, die die Änderungen der Polaritätskonfiguration der Sektoren steuert, so dass der Elektromotor ermöglicht, dass das Gebläse innerhalb eines Atemschritts eines Patienten schnelle Beschleunigungen und Verlangsamungen erzielt, wobei der Atemschritt aus einer Einatmung und einer Ausatmung besteht;
   **dadurch gekennzeichnet, dass** die Antriebseinheit Mittel beinhaltet, um die gegenelektromotorische Kraft, die von dem Elektromotor erzeugt wird, wahrzunehmen, und dass die Antriebseinheit konfiguriert ist, um die Polaritätskonfiguration der Sektoren zu ändern, wenn die gegenelektromotorische Kraft den Nullwert erreicht.

2. Gerät (1) gemäß Anspruch 1, wobei der Stator (8) mindestens drei Sektoren (8a, 8b und 8c) aufweist, wobei jeder der Sektoren an einen Schalter (HA, HB oder HC) angeschlossen ist, der an das positive Kontaktstück der Stromversorgung angeschlossen ist, und wobei jeder der Sektoren an einen Schalter (LA, LB oder LC) angeschlossen ist, der an das negative Kontaktstück der Stromversorgung angeschlossen ist, damit einer der Drehungsschritte des Rotors erlangt wird, wenn die Antriebseinheit (36) durch Anschließen des ersten Sektors (8a) an das positive Kontaktstück, Anschließen des zweiten Sektors (8b) an das negative Kontaktstück und Einstellen des dritten Sektors (8c), der nicht an ein Kontaktstück der Stromversorgung angeschlossen ist, die Spannung auf den Stator anwendet, wodurch ermöglicht wird, die gegenelektromotorische Kraft des Motors zwischen dem dritten Sektor und dem negativen Kontaktstück zu messen, wobei die Antriebseinheit die Polaritätskonfiguration der Sektoren ändert, wenn die gegenelektromotorische Kraft den Nullwert erreicht.

3. Gerät (1) gemäß Anspruch 2, wobei die angewandte Spannung eine Pulsweitenmodulation ist, wobei die Antriebseinheit einen der Sektoren des Stators (8a, 8b oder 8c) während einer ersten Zeitdauer an das positive Kontaktstück anschließt, und dann während einer zweiten Zeitspanne denselben Sektor von dem positiven Kontaktstück trennt und ihn an das negative Kontaktstück anschließt, so dass im Fall einer Verlangsamung des Motors der erzeugte Strom zu dem negativen Kontaktstück geleitet wird, wodurch eine schnelle Verlangsamung des Gebläserads (5) bereitgestellt wird.

4. Gerät (1) gemäß Anspruch 2 oder 3, wobei der Stator (8) ein Stator mit drei Sektoren ist und der Rotor ein Dipolmagnet ist, wodurch der Stator Polaritätskonfiguration von sechs Sektoren aufweist, so dass der Rotor eine 360°-Drehung in sechs Drehungsschritten durchführt.

5. Gerät gemäß einem der vorhergehenden Ansprüche, wobei der Stator (8) ein Ringstator ist and wobei jeder der Sektoren (8a, 8b oder 8c) eine Spule ist, die an lediglich einem Draht angeschlossen ist.

6. Gerät gemäß einem der vorhergehenden Ansprüche, wobei, wenn das Gebläse (33) arbeitet und keine gegenelektromotorische Kraft gemessen wird, die Antriebseinheit (36) die angewandte Spannung fixiert und die Polaritätskonfiguration der Sektoren (8a, 8b und 8c) nach festgelegter Zeit ändert, wobei die Antriebseinheit diese festgelegte Zeit bei jedem Schritt verringert, bis eine gegenelektromotorische Kraft ermittelt wird, und dann die erforderliche Spannung anwendet und die Polaritätskonfiguration der Sektoren gemäß dem Wert der gegenelektromotorischen Kraft ändert.

7. Gerät gemäß einem der vorhergehenden Ansprüche, das ferner Lager (4a und 4b) und eine Lagerhalterung (93) beinhaltet, und wobei das Gebläserad (5), der Rotor (3) und die Welle (91) des Gebläserads aneinander fixiert sind, wobei die inneren Ringe der Lager an der Welle fixiert sind und die äußeren Ringe der Lager von der Lagerhalterung, die in dem Gerät fixiert ist, gehalten werden, und wobei der Rotor außerhalb des Stators verschoben wird, vorzugsweise in einer gleichen Distanz zu jedem der drei Sektoren des Stators (8a, 8b und 8c), so dass der Stator auf dem Rotor ebenfalls eine axiale Kraft erzeugt, die entlang der Welle ausgerichtet ist, wodurch eine Vorlast auf den Lagern erzeugt wird.

8. Gerät gemäß einem der vorhergehenden Ansprüche, das ferner einen Stromversorgungsregler (29) beinhaltet, der an die Stromversorgungsquelle (30 oder 31) angeschlossen ist, wobei der Stromversorgungsleiter einen Stromsensor (55), einen Komparator (53), einen Belastungswiderstand (54) und ein Mittel zum Einschalten des Belastungswiderstands zwischen der positiven Stromversorgung und dem Boden beinhaltet, wenn der von dem Stromsensor gemessene Strom negativ ist, um diesen Strom in dem Belastungswiderstand durch die Wärmewirkung abzuleiten.

9. Gerät gemäß einem der vorhergehenden Ansprüche, wobei es ausgelegt ist, um an ein Rohr mit einem Durchmesser von weniger als 22 Millimetern angeschlossen zu werden, wobei ein erstes Ende des Rohrs an einen Luftauslass des Gebläses (33) angeschlossen ist und ein zweites Ende des Rohrs an eine Maske angeschlossen ist, in der der Patient atmet.

10. Gerät gemäß einem der vorhergehenden Ansprüche, das ferner Folgendes beinhaltet:

- mindestens ein Mittel (110) zum Ermitteln der Atmungsparameter des Patienten,
- eine Drucksteuereinheit (37) zum Abstimmen des von dem Gebläse gelieferten Drucks auf das Niveau der Maske, und das ein Schätzmodul (100) beinhaltet, das an dem Mittel zum Ermitteln der Atmungsparameter des Patienten angeschlossen ist, damit das Schätzmodul bestimmen kann, wann der Patient einatmet oder ausatmet, und als Reaktion zum Anwenden des Drucks auf die Maske des Patienten während der Einatmung und während der Ausatmung.

11. Gerät gemäß Anspruch 10, wobei das mindestens eine Mittel den Luftfluss des Patienten ermittelt und ihn zu einem Atemschätzer (132) leitet, der den Luftfluss als eine Funktion von Zeit bestimmt und diese Funktion an das Schätzmodul (100) überträgt, das somit den Druck schätzen wird, um ihn gemäß der Luftflussfunktion auf die Maske des Patienten anzuwenden, um die Anstrengung der Lunge des Patienten zu verringern, während während eines Atemschritts der Durchschnittswert des Drucks $P_M$ an der Maske gleich dem Druck der Behandlung beibehalten wird, wobei das Schätzmodul (100) vorzugsweise den Druck $P_M$ an der Maske als eine Funktion von Zeit bestimmt.

12. Gerät gemäß Anspruch 10 oder 11, wobei die Steuereinheit einen nichtflüchtigen Speicher beinhaltet, in den der Krankenhausarzt klinische Einstellwerte (120) eingeben kann, die mindestens den Behandlungsdruck und möglicherweise den gemäß den Atmungsparametern des Patienten anzuwendenden Druck beinhalten, wobei das Schätzmodul den Druck PM gemäß diesen klinischen Einstellwerten und den Atmungsparametern des Patienten bereitstellt.

13. Gerät gemäß Anspruch 12, wobei der Patient Einstellwerte des Patienten (122) in den nichtflüchtigen Speicher eingeben kann, wobei das Schätzmodul (100) den Druck gemäß diesen Einstellwerten des Patienten und den Atmungsparametern des Patienten innerhalb von den klinischen Einstellwerten (120) festgelegten Beschränkungen bereitstellt.

14. Gerät gemäß einem der Ansprüche 10 bis 13, wobei das Schätzmodul (100) bestimmen kann, dass ein Ereignis ($E_1$, $E_2$ oder $E_3$) bei der Atmung des Patienten auftritt, wodurch ermöglicht wird, dass die Drucksteuereinheit (37) das Gebläse (33) mit der anzuwendenden Spannung bereitstellt, um den Druck an der Maske des Patienten abzustimmen.

**15.** Gerät gemäß einem der Ansprüche 10 bis 14, wobei die Mittel (22 und 23) zum Ermitteln der Atmungsparameter des Patienten ermöglichen, dass die Drucksteuereinheit (2) den Luftfluss an der Maske des Patienten berechnet, wobei das Schätzmodul (100) bestimmt, dass ein Ereignis ($E_1$, $E_2$ oder $E_3$) mit den Luftflussparametern oder der Form auftritt.

**16.** Gerät gemäß Anspruch 10 bis 15, wobei das Schätzmodul (100) einen Einatmungsausgang (102) aufweist, an dem das Schätzmodul den Wert des Maskendrucks PM während der Einatmung eingestellt hat, und wobei das Schätzmodul einen Ausatmungsausgang (104) aufweist, an dem das Schätzmodul den Wert des Maskendrucks PM während der Ausatmung eingestellt hat, wobei die Drucksteuereinheit (37) einen Schalter (108) beinhaltet, der gemäß der Atmung des Patienten alternativ an den Einatmungsausgang oder Ausatmungsausgang angeschlossen ist.

**17.** Gerät gemäß Anspruch 10 bis 16, wobei die Mittel zum Ermitteln der Atmungsparameter des Patienten einen Drucksensor (23) zum Wahrnehmen des Drucks an dem ersten Rohrende und einen Drucksensor (22) zum Wahrnehmen des Drucks an dem Ende des Rohrs, das an den Gebläseauslass angeschlossen ist, beinhalten, wobei das Luftflussberechnungsmodul den Luftfluss aus diesen Drücken und aus dem Widerstandskoeffizienten KT des Luftflusses des Rohrs errechnen kann.

**18.** Gerät gemäß Anspruch 10 bis 17, wobei das Gerät ferner ein Startmittel beinhaltet, das, wenn in Betrieb, dem Schätzmodul (100) befiehlt, eine Atmungsaktivität zu ermitteln, wobei das Schätzmodul die Anweisung aussendet, das Gebläse (33) anzuhalten, wenn nach festgelegter Verzögerung keine Aktivität wahrgenommen wird.

**19.** Gerät gemäß einem der vorhergehenden Ansprüche, wobei ein Stromversorgungsregler (29) ein Kommunikationsmodul (65) beinhaltet, das die Daten durch die Drähte der Stromquelle überträgt.

**20.** Gerät gemäß einem der vorhergehenden Ansprüche, wobei das Kommunikationsmodul (65) einen Modulator (50) zur Frequenzumtastung (FSK) beinhaltet, der von den Gerätsensoren oder -elementen ausgesandte binäre Daten in einer Modulation der Frequenz der Spannung umwandelt, die auf eine spannungsgesteuerte Stromquelle (52), die an die externe Stromversorgung angeschlossen ist, angewandt wird, so dass die spannungsgesteuerte Stromquelle die den Daten entsprechende Modulation überträgt, wobei ein FSK-Demodulator die Modulation der Spannungsfrequenz in binäre Daten (61) konvertiert, und sie an die Elemente überträgt, so dass jeder Sensor oder jedes Modul, die an die Stromquelle angeschlossen sind, Informationen empfangen oder übertragen kann.

**21.** Gerät gemäß einem der vorhergehenden Ansprüche, das ferner ein phonisches Isolationsbehältnis (34) beinhaltet, in dem das Gebläse (33) platziert ist, wobei das Gebläserad (5) eine Größe von weniger als 60 mm aufweist und zwischen 15 und 45 Flügel, vorzugsweise 27, beinhaltet, so dass sich das Gebläserad bei einer Geschwindigkeit dreht, die einen Ton von hoher Frequenz erzeugt, wobei ermöglicht wird, dass das Behältnis den Patienten von diesem Geräusch isoliert.

**22.** Gerät gemäß einem der vorhergehenden Ansprüche, wobei das Gerät zur Verwendung für die Behandlung von Atmungsanomalien wie etwa Schnarchen, Atemstillstand oder flacher langsamer Atmung angepasst ist.

**23.** Gerät gemäß einem der Ansprüche 5 bis 22, wobei der Stator (8) durch Bandkerntechnik mit hochgradigem Siliziumstahl von etwa 0,05 mm Dicke erlangt wird.

**Revendications**

**1.** Un appareil (1) pour assister la respiration d'un patient en amenant de l'air à ce patient par l'intermédiaire d'un masque, comprenant un dispositif de soufflage d'air (33) dans lequel l'hélice (5) est entraînée en rotation par un moteur électrique comprenant un rotor (3) et un stator (8), ledit stator ayant au moins trois secteurs (8a, 8b et 8c), la rotation du rotor étant permise par des changements de la polarité des secteurs, la configuration de polarité de chaque secteur constituant une étape de la rotation du rotor, ledit appareil comprenant une unité d'entraînement (36) contrôlant des changements de la configuration de polarité des secteurs de telle sorte que le moteur électrique permette au dispositif de soufflage de parvenir à des accélérations et décélérations rapides au sein d'une étape de respiration du patient, ladite étape de respiration consistant en une inspiration et une expiration ; **caractérisé en ce que** ladite unité d'entraînement comprend des moyens pour capter la force contre-électromotrice générée par le moteur électrique et **en ce que** ladite unité d'entraînement est configurée pour changer la configuration de polarité des secteurs lorsque la force contre-électromotrice atteint la valeur zéro.

**2.** L'appareil (1) selon la revendication 1, dans lequel ledit stator (8) a au moins trois secteurs (8a, 8b et 8c), chacun desdits secteurs étant connecté à un commutateur (HA, HB, ou HC) connecté au plot positif de l'alimentation électrique et chacun desdits secteurs étant connecté à un commutateur (LA, LB ou LC) connecté au plot négatif de l'alimentation électrique, afin que l'une des étapes de rotation du rotor soit obtenue lorsque ladite unité d'entraînement (36) applique la tension sur le stator en connectant le premier secteur (8a) audit plot positif, en connectant le deuxième secteur (8b) au plot négatif et en réglant le troisième secteur (8c) pour qu'il ne soit pas connecté à un plot d'alimentation électrique, ce qui permet ainsi de mesurer la force contre-électromotrice du moteur entre le troisième secteur et le plot négatif, ladite unité d'entraînement changeant la configuration de polarité des secteurs lorsque la force contre-électromotrice atteint la valeur zéro.

**3.** L'appareil (1) selon la revendication 2, dans lequel ladite tension appliquée est une modulation d'impulsions en largeur, l'unité d'entraînement connectant l'un desdits secteurs de stator (8a, 8b ou 8c) au plot positif pendant un premier laps de temps puis, durant une deuxième période, déconnectant le même secteur du plot positif et le connectant au plot négatif, de sorte qu'en cas de décélération du moteur le courant généré est envoyé au plot négatif, fournissant ainsi une décélération rapide de l'hélice (5).

**4.** L'appareil (1) selon la revendication 2 ou 3, dans lequel ledit stator (8) est un stator à trois secteurs et ledit rotor est un aimant dipolaire, ledit stator ayant ainsi une configuration à six polarités de secteurs de sorte que le rotor effectue une rotation sur 360° en six étapes de rotation.

**5.** L'appareil selon n'importe laquelle des revendications précédentes, dans lequel ledit stator (8) est un stator torique et dans lequel chacun desdits secteurs (8a, 8b ou 8c) sont des bobines connectées avec un fil seulement.

**6.** L'appareil selon n'importe laquelle des revendications précédentes, dans lequel lorsque le dispositif de soufflage (33) fonctionne et qu'il n'est pas mesuré de force contre-électromotrice, ladite unité d'entraînement (36) fixe la tension appliquée et change la configuration de polarité des secteurs (8a, 8b, et 8c) après un temps donné, ladite unité d'entraînement diminuant ce temps donné à chaque étape jusqu'à ce qu'une force contre-électromotrice soit détectée, puis appliquant la tension requise et changeant la configuration de polarité des secteurs en fonction de la valeur de force contre-électromotrice.

**7.** L'appareil selon n'importe laquelle des revendications précédentes, comprenant en outre des roulements (4a et 4b) et un support de roulement (93), et dans lequel ladite hélice (5), ledit rotor (3) et l'arbre (91) de ladite hélice sont fixés ensemble, ladite bague interne des roulements étant fixée audit arbre et les bagues externes desdits roulements étant maintenues par le support de roulement, lequel est fixé dans l'appareil, et ledit rotor étant décalé à l'extérieur du stator, de façon préférentielle à une distance égale de chacun des trois secteurs de stator (8a, 8b et 8c), de sorte que le stator génère également sur le rotor une force axiale orientée le long dudit arbre, générant ainsi une précharge sur les roulements.

**8.** L'appareil selon n'importe laquelle des revendications précédentes, comprenant en outre un gestionnaire d'alimentation électrique (29) connecté à la source d'alimentation électrique (30 ou 31), ledit gestionnaire d'alimentation électrique comprenant un capteur de courant (55), un comparateur (53), une résistance de charge (54) et un moyen pour mettre en marche la résistance de charge entre l'alimentation électrique positive et la terre lorsque le courant mesuré par ledit capteur de courant est négatif, afin de dissiper ce courant dans ladite résistance de charge par effet thermique.

**9.** L'appareil selon n'importe lesquelles des revendications précédentes, lequel est conçu pour être connecté à un tube d'un diamètre inférieur à 22 millimètres, une première extrémité du tube étant connectée à la sortie d'air du dispositif de soufflage (33) et une deuxième extrémité du tube étant connectée à un masque dans lequel respire le patient.

**10.** L'appareil selon n'importe lesquelles des revendications précédentes comprenant en outre:

> au moins un moyen (110) pour détecter les paramètres de respiration du patient,
> une unité de commande de pression (37) pour ajuster la pression amenée par ledit dispositif de soufflage au niveau dudit masque, et comprenant un module d'estimation (100) connecté au moyen pour détecter les paramètres de respiration du patient, afin que le module d'estimation soit en mesure de déterminer lorsque le patient inspire ou expire et en réponse la pression à appliquer au masque du patient, durant l'inspiration et durant l'expiration.

**11.** L'appareil selon la revendication 10, dans lequel ledit au moins un moyen détecte le débit d'air du patient et l'envoie à un estimateur de respiration (132), lequel détermine le débit d'air en fonction du temps et transmet cette fonction audit module d'estimation (100), lequel estimera ainsi la pression à appliquer au masque du patient selon la fonction de débit d'air, afin de diminuer l'effort du poumon du patient tout en maintenant, durant une étape de respiration, la valeur moyenne de la pression au masque $P_M$ égale à la pression de traitement, le module d'estimation (100) déterminant de façon préférentielle la pression $P_M$ au masque en fonction du temps.

**12.** L'appareil selon la revendication 10 ou 11, dans lequel l'unité de commande comprend une mémoire non volatile dans laquelle le clinicien peut entrer des réglages cliniques (120) comprenant au moins la pression de traitement et éventuellement la pression à appliquer en fonction des paramètres de respiration du patient, ledit module d'estimation fournissant la pression PM en fonction de ces réglages cliniques et des paramètres de respiration du patient.

**13.** L'appareil selon la revendication 12, dans lequel le patient peut entrer des réglages patient (122) dans ladite mémoire non volatile, ledit module d'estimation (100) fournissant la pression en fonction de ces réglages patient et des paramètres de respiration du patient dans des limites données par les réglages clinicien (120).

**14.** L'appareil selon n'importe laquelle des revendications 10 à 13, dans lequel le module d'estimation (100) est en mesure de déterminer qu'il se produit un événement ($E_1$, $E_2$ ou $E_3$) dans la respiration du patient, ce qui permet ainsi à ladite unité de commande de pression (37) de fournir au dispositif de soufflage (33) la tension à appliquer pour ajuster la pression au masque du patient.

**15.** L'appareil selon n'importe laquelle des revendications 10 à 14, dans lequel lesdits moyens (22 et 23) pour détecter les paramètres de respiration du patient permettent à l'unité de commande de pression (2) de calculer le débit d'air au masque du patient, ledit module d'estimation (100) déterminant qu'il se produit un événement ($E_1$, $E_2$ ou $E_3$) avec la forme ou les paramètres du débit d'air.

**16.** L'appareil selon les revendications 10 à 15, dans lequel ledit module d'estimation (100) a une sortie d'inspiration (102) où ledit module d'estimation règle la valeur PM de pression au masque durant l'inspiration, et dans lequel ledit module d'estimation a une sortie d'expiration (104) où ledit module d'estimation règle la valeur PM de pression au masque durant l'expiration, ladite unité de commande de pression (37) comprenant un commutateur (108) qui est connecté de façon alternative à la sortie d'inspiration ou à la sortie d'expiration en fonction de la respiration du patient.

**17.** L'appareil selon les revendications 10 à 16, dans lequel le moyen pour détecter les paramètres de respiration du patient comprennent un capteur de pression (23) destiné à capter la pression à ladite première extrémité de tube et un capteur de pression (22) destiné à capter la pression à l'extrémité du tube connectée à la sortie du dispositif de soufflage, ledit module de calcul de débit d'air étant en mesure de calculer le débit d'air d'après ces pressions et d'après le coefficient de résistance au débit d'air du tube KT.

**18.** L'appareil selon les revendications 10 à 17, dans lequel l'appareil comprend en outre un moyen de mise en marche qui, lorsqu'il est activé, donne au module d'estimation (100) l'ordre de détecter une activité de respiration, ledit module estimateur envoyant l'instruction d'arrêter le dispositif de soufflage (33) s'il n'est capté aucune activité après un délai donné.

**19.** L'appareil selon n'importe laquelle des revendications précédentes, dans lequel un gestionnaire d'alimentation électrique (29) comprend un module de communication (65) qui transmet les données par l'intermédiaire des fils de la source d'alimentation.

**20.** L'appareil selon n'importe laquelle des revendications précédentes, dans lequel ledit module de communication (65) comprend un modulateur (50) à modulation par déplacement de fréquence (MDF) qui transforme les données binaires envoyées par les capteurs ou éléments de l'appareil en une modulation de la fréquence de la tension appliquée sur une source de courant commandée en tension (52), connectée à l'alimentation électrique externe, de sorte que la source de courant commandée en tension transmette la modulation correspondant aux données, et un démodulateur MDF qui convertit la modulation de fréquence de tension en données binaires (61) et les transmet aux éléments, de sorte que chaque capteur ou module connecté à la source d'alimentation est en mesure de recevoir ou de transmettre des informations.

**21.** L'appareil selon n'importe lesquelles des revendications précédentes, comprenant en outre un boîtier d'isolation phonique (34) dans lequel est placé le dispositif de soufflage (33), ladite hélice (5) ayant une dimension inférieure à 60 mm et comprenant entre 15 et 45 lames, de façon préférentielle 27, de sorte que l'hélice tourne à une vitesse qui génère un son à une fréquence haute, ce qui permet audit boîtier d'isoler le patient de ce bruit.

22. L'appareil selon n'importe laquelle des revendications précédentes, ledit appareil étant adapté pour être utilisé pour le traitement d'anomalies respiratoires, telles que ronflement, apnées, ou hypopnées.

23. L'appareil selon les revendications 5 à 22, dans lequel ledit stator (8) est obtenu par une technique des noyaux magnétiques enroulés avec un acier au silicium fin de qualité, d'environ 0,05 mm d'épaisseur.

FIG. 1

FIG. 2

FIG. 3

FIG. 4a

FIG. 4b

**FIG. 5**

$$VM = \frac{VPOWER}{2} + Vbemf$$

**FIG.6**

PWM on

On

Off

PWM period

$$PWM = \frac{PWM\ "on"}{PWM\ "Period"}$$

FIG. 7

HA    HB    HC

LA    LB    LC

26

38

95

FIG.8

EP 1 485 155 B1

PWM ORDER

YES ◄—— MOTOR STOPPED ? ——► NO

INITIALIZE TIME BETWEEN STEPS
FIXE PWM VALUE

BEMF
DETECTED
?

NO

DECREASE TIME
BETWEEN STEPS

YES

APPLY REQUIRED
PWM VALUE

FIG. 9

FIG. 10

FIG. 11

FIG. 12

FIG. 13

FIG. 14

FIG. 15

FIG. 16a

FIG. 16b

EP 1 485 155 B1

FIG. 17